# EUROPÄISCHE PATENTANMELDUNG

(11) **EP 3 785 622 A1**
(43) Veröffentlichungstag der Anmeldung: **03.03.2021**
(21) Anmeldenummer: 19193595.6
(22) Anmeldetag: 26.08.2019
(51) Int. Cl.: A61B 5/0478, A61B 5/00, A61B 5/055

(54) **VORRICHTUNG ZUM MESSEN VON EEG-SIGNALEN**

(71) Anmelder: Siemens Healthcare GmbH, 91052 Erlangen (DE)
(72) Erfinder: Popescu, Stefan, 91056 Erlangen (DE)

(57) **Zusammenfassung**

Die Erfindung betrifft eine Vorrichtung zum Messen von EEG-Signalen in einem Ohr einer Untersuchungsperson, aufweisend:
- ein Gehäuse,
- zwei auf einer Außenseite des Gehäuses angeordneten Signalelektroden (3) zur Aufnahme von EEG-Signalen von einer Innenfläche des Ohres,
wobei in dem Gehäuse vorgesehen sind:
- ein Verstärker mit zumindest zwei Eingängen zur Verstärkung der mit den Signalelektroden aufgenommenen EEG-Signalen,
- eine Sende-/Empfangseinheit, die ausgebildet ist, EEG-Daten nach der Verstärkung durch den Verstärker drahtlos an eine zentrale Verarbeitungseinheit zu übertragen,
- zwei Signalleitungen, über die die EEG Signale der Signalelektroden an den Verstärker übertragen werden, wobei jede der zwei Signalleitungen nicht länger als 2 cm ist, vorzugsweise nicht länger als 1 cm.

## Beschreibung

Bei Untersuchungen in bildgebenden Vorrichtungen wie beispielsweise Magnetresonanzanlagen, MR-Anlagen, besteht das Problem, dass die Untersuchungspersonen teilweise unter Klaustrophobie oder anderen Untersuchungsängsten leiden, so dass diese Untersuchungspersonen entweder nicht untersucht werden können oder eine Sedierung notwendig ist, um die Untersuchung durchführen zu können. Es ist daher wichtig, ein Unbehagen, Panik oder Angst bei der Untersuchungsperson zu detektieren, um darauf reagieren zu können. Bei den meisten Untersuchungsmöglichkeiten kann die Bedienperson oder ein Familienangehöriger während der Untersuchung nicht im Untersuchungsraum in der Nähe der Untersuchungsperson bleiben. Im Falle von MR-Untersuchungen wird die Sprachkommunikation zwischen dem Patienten und der Bedienperson außerhalb des Untersuchungsraums über Mikrofone und Lautsprecher beeinträchtigt durch den Lärm, den die MR-Untersuchung erzeugt. Ebenso ist es möglich, dass der Patient und die Bedienperson unterschiedliche Sprachen sprechen. Weiterhin ist es möglich, dass die Untersuchungspersonen nicht sprechen können aufgrund gesundheitlicher Einschränkungen wie beispielsweise einer Lähmung oder einem Schlaganfall.

Insgesamt besteht bei Untersuchungen der Bedarf, physiologische Parameter der Untersuchungsperson während der Untersuchung zu messen, um Komplikationen zu vermeiden. Bei einigen klinischen Studien wie beispielsweise der funktionellen Bildgebung ist es auch notwendig, zuverlässig zu erkennen, ob die Untersuchungsperson schläft. Ebenso kann es hilfreich sein, anhand von physiologischen Parametern die Untersuchungsperson genau zu identifizieren.

Bei einer MR-Anlage hat die Untersuchungsperson üblicherweise einen Gummiball in der Hand, den sie im Notfall drücken kann. Die richtige Positionierung dieses Gummiballs bei der Positionierung der Untersuchungsperson ist jedoch zeitaufwendig. Weiterhin sind Personen ohne Bewusstsein nicht in der Lage, diesen Ball zu drücken. Zusätzlich werden Videokameras zur Überwachung der Untersuchungspersonen verwendet, jedoch kann es bei MR-Anlagen schwierig sein, daraus genaue Informationen über die Untersuchungsperson zu ziehen durch die Anwesenheit der verwendeten Spulen, die die Sicht verdecken können.

Weiterhin sind EEG-Vorrichtungen (Elektroenzephalogramm-Vorrichtung) im Ohr bekannt, bei denen Elektroden im Gehörgang und eine Referenzelektrode am Ohrläppchen die Gehirnwellen messen können, die von der neuronalen Aktivität des Gehirns erzeugt werden. Hierbei werden Elektroden verwendet, die am Kopf in der Nähe des Temporallappens befestigt werden. Der Temporallappen ist einer der vier Hauptlappen des zerebralen Kortex des Gehirns, wobei dieser bei der Verarbeitung von sensorischen Anregungen involviert ist, die sich bei der Wahrnehmung von Sprache oder Bildern ergeben.

Eine Herausforderung bei der Messung von EEG-Signalen besteht darin, dass die Amplitude dieser Signale im Bereich zwischen 10 und 100 µV liegt, und somit die Messinstrumente sensitiv gegenüber externen parasitären Interferenzen sind, die durch Induktion oder Streukapazität in den EEG-Leitungen induziert werden, die die Elektroden mit dem EEG-Verstärker verbinden. Auch wenn die Induktionsfläche minimiert wird, indem alle Elektroden nahe nebeneinander am oder im Ohr positioniert werden, ist die kapazitive Kopplung des Untersuchungskörpers oder der EEG-Kabel durch Streukapazitäten der elektrischen Komponenten ein Problem. Aus diesem Grund wird neben einer Referenzelektrode auf dem Ohrläppchen eine weitere Erdungselektrode auf dem Kinn platziert. Dadurch wird eine Interferenz reduziert. Wenn derartige EEG-Systeme in Verbindungen mit bildgebenden Vorrichtungen verwendet werden, besteht das Risiko von Verletzungen durch induzierte Gleichtaktspannungen entlang der Kabel, die die Elektroden mit einen EEG-Verstärker verbinden, der das EEG-Signal verstärkt. Dies gilt insbesondere in Anwesenheit der starken und variierenden elektromagnetischen Felder, die bei MR-Anlagen verwendet werden.

Es ist daher eine Aufgabe der vorliegenden Erfindung, ein EEG-System bereitzustellen, das die oben erwähnten Nachteile vermeidet und insbesondere in Kombination mit MR-Untersuchungen verwendet werden kann.

Diese Aufgabe wird durch die Merkmale der unabhängigen Ansprüche gelöst. In den abhängigen Ansprüchen sind weitere Ausführungsformen beschrieben.

Es wird eine Vorrichtung zum Messen von EEG-Signalen in einem Ohr einer Untersuchungsperson bereitgestellt, wobei die Vorrichtung ein Gehäuse aufweist und zwei auf einer Außenseite des Gehäuses angeordnete Signalelektroden zur Aufnahme der EEG-Signale von einer Innenfläche des Ohrs. In dem Gehäuse ist weiterhin ein Verstärker vorgesehen mit mindestens zwei Eingängen zur Verstärkung der mit den Signalelektroden aufgenommen EEG-Signale. Im Gehäuse ist weiterhin eine Sende- und Empfangseinheit vorgesehen, die ausgebildet ist, EEG-Daten nach der Verstärkung durch den Verstärker drahtlos an eine zentrale Verarbeitungseinheit zu übertragen. Weiterhin sind zwei Signalleitungen vorgesehen, über die die EEG-Signale der Signalelektroden an den Verstärker übertragen werden, wobei jede der zwei Signalleitungen nicht länger als 2 cm ist, vorzugsweise nicht länger 1 cm ist.

Durch die Verwendung der drahtlosen Sende- und Empfangseinheit ist es möglich, die Signalleitungen zur Leitung der EEG-Signale vor der Verstärkung sehr kurz zu halten. Diese Signalleitungen und die darin übertragenen Signale sind sehr störempfindlich aufgrund der geringen Amplitude. Diese Signalleitungen mit den EEG-Signalen vor der Verstärkung sind nicht länger als 2 cm, vorzugsweise nicht länger als 1 cm.

Damit ist diese neue Lösung sehr robust gegenüber externer elektromagnetischer Interferenz, EMI, die beispielsweise bei großen Leiterschleifen vorkommen oder durch die kapazitive Kopplung mit den langen Drähten. Störquellen wie beispielsweise Leistungsversorgungsleitungen mit 50Hz, die mit langen EEG Drähten entstehen, werden somit vermieden.

Insgesamt hat das Gehäuse vorzugsweise Außenmaße, die 5 cm, bevorzugt 3 cm nicht überschreiten. Damit werden die durch ein HF Feld in einer MR Anlage induzierten Spannungen vermieden.

Vorzugsweise weist das Gehäuse einen ersten Gehäuseabschnitt und einen zweiten Gehäuseabschnitt auf, die lösbar miteinander verbindbar sind. Hierbei kann der erste Gehäuseabschnitt die beiden Signalelektroden aufweisen und der zweite Gehäuseabschnitt den Verstärker und die Sende-/Empfangseinheit.

In dieser Ausführungsform mit einer zumindest zweiteiligen Ausführung des Gehäuses weist der erste Gehäuseabschnitt im Wesentlichen nur die Signalelektroden auf, wobei die Verarbeitung der EEG-Signale zu den EEG-Daten für die Übertragung im zweiten Gehäuseabschnitt erfolgt. Dadurch ist keine teure Technik im ersten Gehäuseabschnitt notwendig, dieser kann als Wegwerfprodukt ausgeführt sein, so dass die Vorrichtung einfach bei verschiedenen Anwendern angewendet werden kann durch Verwendung eines neuen ersten Gehäuseabschnitts.

Für die Verbindung kann der erste Gehäuseabschnitt ein erstes Verbindungselement und der zweite Gehäuseabschnitt ein zweites, komplementäres Verbindungselement aufweisen. Diese beiden Verbindungselemente sind ausgebildet, den ersten Gehäuseabschnitt und den zweiten Gehäuseabschnitt lösbar wiederholt miteinander zu verbinden. Das erste Verbindungselement am ersten Gehäuseabschnitt kann zwei Kontaktelemente aufweisen, die in Verbindung mit den zwei Signalelektroden stehen, wobei das zweite Verbindungselement zwei weitere Kontaktelemente aufweisen kann, die jeweils in elektrischer Verbindung mit dem Verstärker stehen. Weiterhin kann jedes der zwei weiteren Kontaktelemente im verbundenen Zustand der beiden Gehäuseabschnitte jeweils mit einem der zwei Kontaktelemente verbunden sein.

Durch die zwei Kontaktelemente und die zwei weiteren Kontaktelemente wird sichergestellt, dass die von den Signalelektroden aufgenommenen EEG-Signale im verbundenen Zustand an den Verstärker geleitet werden können.

Das erste Verbindungselement kann hierbei als Kontaktbuchse und das zweite komplementäre Verbindungselement als Kontaktstift ausgebildet sein. Hierbei sind dann die beiden weiteren Kontaktelemente auf dem Kontaktstift ausgebildet, und die Kontaktelemente des ersten Verbindungselements sind als Kontaktringe ausgebildet, in die der Kontaktstift im verbundenen Zustand eingeführt ist und die die auf dem Kontaktstift ausgebildeten weiteren Kontaktelemente kontaktieren.

In dieser Ausführungsform weist der Kontaktstift die beiden Kontaktelemente, vorzugsweise auf der Außenseite, auf. Diese Kontaktelemente kontaktieren dann die Kontaktringe, wobei ein einzelnes Kontaktelement jeweils einen der beiden Kontaktring kontaktiert.

Weiterhin kann in dem Gehäuse zusätzlich ein Signalprozessor vorgesehen sein, der ausgebildet ist, ein verstärktes EEG-Signal nach Verstärkung am Verstärker zu empfangen, und der das verstärkte EEG-Signal filtern und einer Analog-Digital-Wandlung unterziehen kann. Vorzugsweise ist dieser Signalprozessor ebenfalls in dem zweiten Gehäuseabschnitt vorgesehen, in dem der Verstärker und die Sende-/Empfangseinheit angeordnet sind. Der Signalprozessor kann die EEG-Daten, die von der Sende-/Empfangseinheit verschickt werden, erzeugen.

Zusätzlich kann die Vorrichtung einen optischen Sensor aufweisen, der ausgebildet ist, den pulsierenden Blutfluss im Ohr und damit die Herztätigkeit der Untersuchungsperson zu detektieren.

Vorzugsweise hat das Gehäuse Ausmaße, die 5 cm oder 3 cm nicht überschreiten.

Weiterhin hat die Vorrichtung bevorzugt nur zwei Signalelektroden, eine dritte Elektrode als Referenz ist nicht notwendig. In jedem Ohr werden damit nur zwei EEG Signale aufgenommen.

Weiterhin ist ein System zur Verarbeitung von EEG-Signalen vorgesehen, das eine Vorrichtung wie oben erläutert wurde oder nachfolgend im Detail beschrieben wird, aufweist und eine zentrale Verarbeitungseinheit. Diese ist dazu in der Lage, die von der Sende-/Empfangseinheit drahtlos übertragenen EEG-Daten zu empfangen und weiterzuverarbeiten.

Das System kann jeweils zwei Vorrichtungen für die EEG-Signaldetektion aufweisen wie oben beschrieben, wobei eine Vorrichtung für jedes Ohr der Untersuchungsperson vorgesehen ist. Hierbei kann die zentrale Verarbeitungseinheit so ausgebildet sein, dass sie ein Referenzzeitsignal für die beiden Vorrichtungen erzeugt, mit dem die EEG-Daten der beiden Vorrichtungen zeitlich synchronisiert werden. Dieses Referenzzeitsignal kann beispielsweise an die beiden Vorrichtungen drahtlos übertragen werden, damit beide Vorrichtungen mit der gleichen Zeit arbeiten.

Die zentrale Verarbeitungseinheit kann hierbei eine zentrale Steuereinheit einer MR-Anlage sein.

Die zentrale Verarbeitungseinheit kann weiterhin ausgebildet sein, anhand der EEG-Daten verschiedene Informationen aus den EEG-Daten zu extrahieren. Beispielsweise kann die Verarbeitungseinheit ausgebildet sein, anhand der EEG-Daten zu detektieren, ob eine Sprachnachricht, die an die Untersuchungsperson gerichtet wurde, von der Untersuchungsperson im Gehirn richtig verarbeitet wurde. Weiterhin kann die Verarbeitungseinheit ausgebildet sein, aus den EEG-Daten unausgesprochene Gedanken zu extrahieren und in eine Sprachnachricht umzuwandeln. Ebenso ist es möglich, dass die Verarbeitungseinheit anhand der EEG-Daten detektiert, ob die Untersuchungsperson Angst hat oder sonstiges Unbehagen verspürt. Zusätzlich kann detektiert werden, ob die Untersuchungsperson das Bewusstsein verliert, ob die Untersuchungsperson schläft, oder es ist möglich, die Identität einer Untersuchungsperson anhand der EEG-Daten zu detektieren und zu bestimmen.

Die oben dargelegten Merkmalen und die nachfolgend beschriebenen Merkmale können nicht nur in den entsprechend explizit dargelegten Kombinationen verwendet werden, sondern auch in weiteren Kombinationen, sofern es nicht explizit anders erwähnt ist.

Die Erfindung wird nachfolgend unter Bezugnahme auf die beiliegenden Zeichnungen näher erläutert.
Fig. 1 zeigt hierbei schematisch eine EEG-Anordnung nach dem Stand der Technik mit langen Leitungskomponenten und mehr als zwei Elektroden.
Fig. 2 zeigt schematisch eine erfindungsgemäße Anordnung zur Aufnahme von EEG-Daten.
Fig. 3 zeigt schematisch die Anordnung eines Teils der EEG-Vorrichtung nach Fig. 2 im Ohr einer Untersuchungsperson.
Fig. 4 zeigt schematisch eine Teilansicht eines Gehäuseabschnitts der Vorrichtung von Fig. 2.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Weiterhin sind die Figuren schematische Darstellungen verschiedener Ausführungsformen der Erfindung. In den Figuren dargestellte Elemente sind nicht notwendigerweise maßstabsgetreu dargestellt. Die in den Figuren dargestellten Elemente sind vielmehr derart wiedergegeben, dass ihre Funktion und ihr Zweck für den Fachmann verständlich werden. Die in den Figuren zwischen den funktionellen Einheiten oder sonstigen Elementen dargestellten Verbindungen können auch als indirekte Verbindung implementiert werden, wobei eine Verbindung drahtlos oder drahtgebunden sein kann. Funktionelle Einheiten können als Hardware, Software, Firmware oder eine Kombination daraus implementiert werden.

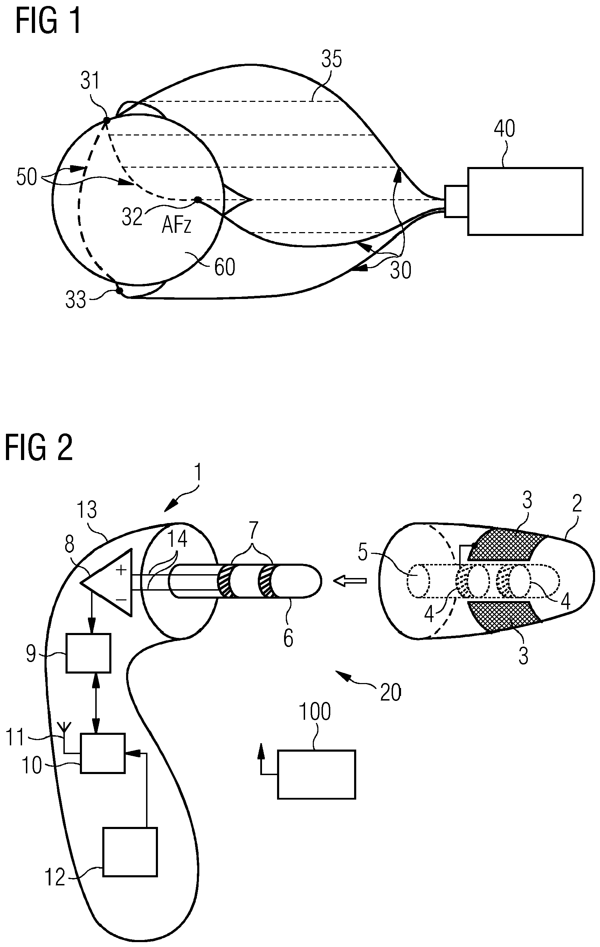

Fig. 1 zeigt eine EEG-Vorrichtung nach dem Stand der Technik, wobei eine Untersuchungsperson 60 dargestellt ist, mit verschiedenen Elektroden 31, 32 und 33, wobei insbesondere die Positionen der Referenzelektroden dargestellt sind. Diese Elektroden sind über Signalleitungen 30 an einen Verstärker 40 angeschlossen, der die detektierten EEG-Signale verstärkt, bevor sie weiterverarbeitet werden. Durch die Position der verschiedenen Elektroden am Kinn bzw. hinter den Ohren oder am Ohr ergibt sich eine Induktionsfläche 35 mit einem gestrichelten elektrischen Leitungsweg 50 durch das Gehirngewebe. Insbesondere bei Verwendung eines derartigen Systems in Verbindung mit MR-Systemen besteht die Gefahr erstens für den Patienten bzw. die Untersuchungsperson, dass Induktionsströme induziert werden, zweitens werden die schwachen EEG-Signale durch die HF-Felder bzw. sich ändernden Gradientenfelder gestört.

Fig. 2 zeigt nun eine erfindungsgemäße Vorrichtung 20, die im dargestellten Fall zweistückig ausgebildet ist. Die zweistückige Ausführung ist insbesondere vorteilhaft bei wiederholter Verwendung der Vorrichtung bei verschiedenen Untersuchungspersonen. Grundsätzlich kann sie jedoch auch einstückig ausgebildet sein. Wie nachfolgend erläutert wird, wird durch die verwendete Geometrie und eine drahtlose Übertragung der schon verstärkten EEG-Signale erreicht, dass erstens die gesamte Vorrichtung sehr kompakt ist, beispielsweise kleiner als 5 cm oder kleiner als 3 cm, und dass insbesondere sehr kurze Signalleitungen zwischen den EEG-Signalelektroden und dem zugehörigen Verstärker verwendet werden können. Diese Signalleitungen können vorzugsweise kürzer sein als 2 cm oder kürzer als 1 cm.

Wie in Fig. 2 zu erkennen ist, weist die Vorrichtung 20 einen ersten Gehäuseabschnitt 2 und einen zweiten Gehäuseabschnitt 1 auf, die lösbar miteinander verbunden werden können. Der zweite Gehäuseabschnitt weist zwei Signalelektroden 3 auf, mit denen bei Einführung in ein Ohr die EEG-Signale detektiert werden können. Jede der Signalelektroden 3 ist mit Kontaktelementen 4 verbunden, die als Kontaktringe ausgebildet sind. Jede der Signalelektroden 3 ist jeweils mit einem Kontaktring 4 verbunden. Der zweite Gehäuseabschnitt 2 weist weiterhin eine Öffnung oder Buchse 5 zur Aufnahme eines komplementären Verbindungselements 6 auf. Auf diesem Verbindungselement 6 sind die weiteren Kontaktelemente 7 vorgesehen, die bei Verbindung der beiden Gehäuseabschnitte, d.h. bei Einführung des Kontaktstifts 6 in die Buchse oder Öffnung 5, mit jeweils einem der Kontaktringe 4 in Verbindung. Jedes Kontaktelement 4 steht in Kontakt mit einem weiteren Kontaktelement 7.

Jedes der weiteren Kontaktelemente 7 ist über eine Signalleitung 14 mit einem Verstärker 8 verbunden, wobei jede Signalleitung mit einem Signaleingang des Verstärkers verbunden ist. Der Verstärker ist ein Verstärker mit hoher Eingangsimpedanz mit eigener Vorspannung. Der Ausgang des Verstärkers 8 wird einem Signalprozessor 9, vorzugsweise einem digitalen Signalprozessor, zugeführt, der die Funktion der Analog-Digital-Wandlung und der Signalvorverarbeitung implementiert. Die Signalvorverarbeitung kann eine digitale Filterung beinhalten zur Eliminierung von Rauschen außerhalb der Bandbreite der EEG-Signale, die üblicherweise zwischen 1 Hz und weniger und ungefähr 70 Hz liegt. Weiterhin können weitere optionale Verarbeitungsschritte wie eine Signalmittelung oder eine Spektralanalyse durch den Signalprozessor durchgeführt werden. Die sich ergebenden digitalen Daten, die EEG-Daten, können weiterhin in Datenpakete segmentiert werden und an eine drahtlose Sende- und Empfangseinheit 10 geschickt werden, die eine bidirektionale Kommunikation mit einer zentralen Verarbeitungseinheit 100 vornimmt, wobei schematisch die HF-Antenne 11 für die drahtlose Übertragung dargestellt ist. Die drahtlose Sende-/Empfangseinheit kann beispielsweise Standardkommunikationsprotokolle wie beispielsweise Bluetooth, Zigbee, ANT oder BT Low Energy unterstützen. Weiterhin ist eine Energiequelle oder Batterie 12 vorgesehen, die vorzugsweise wieder aufladbar ist, beispielsweise eine Lithium-Ionen-Batterie oder ein Kondensator mit hoher Speicherkapazität. Die Energiequelle stellt die Energie bereit, die für die Vorrichtung für einen Betrieb von einer Zeit von beispielsweise mehreren Stunden notwendig ist. Die Vorrichtung kann beispielsweise wieder aufladbar sein durch Positionierung der Vorrichtung in einem weiteren nicht dargestellten Gehäuse, durch das eine drahtlose Energieübertragung für die Aufladung der Batterie möglich ist. Derartige drahtlose Ladesysteme sind aus dem Mobilfunkbereich für Mobiltelefone bekannt.

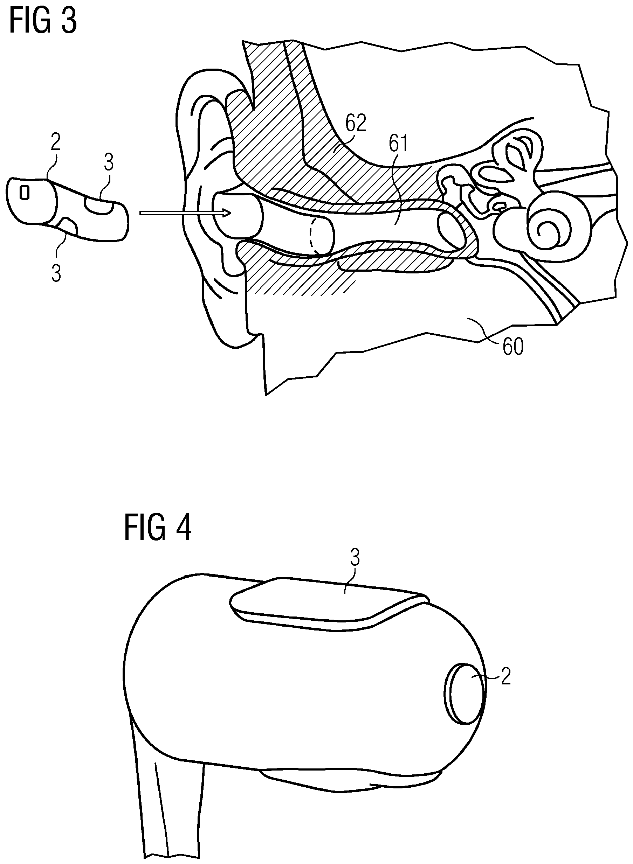

Die äußere Form 13 des Gehäuseabschnitts 1 ist variabel, während die Geometrie des Gehäuseabschnitts 2 derart ausgebildet ist, dass dieser Gehäuseabschnitt komfortabel und passgenau in den Gehörgang eingeführt werden kann. Dies ist genauer in Fig. 3 dargestellt, wo die Positionierung des Gehäuseabschnitts 2 mit den Elektroden 3 relativ zu dem Ohr und Gehörgang der Untersuchungsperson 60 dargestellt ist. Vorzugsweise wird der Gehäuseabschnitt 2 nicht mehr als 10 mm in den Gehörgang 61 eingeführt, insbesondere nicht bis in den Bereich, wo der Gehörgang 61 von Knochen 62 umgeben ist.

In Fig. 2 wurde das erste Verbindungselement als Buchse 5 und das komplementäre Verbindungselement 6 als Kontaktstift dargestellt. Es ist jedoch auch möglich die Buchse am Gehäuseabschnitt 1 und den Kontaktstift am Gehäuseabschnitt 2 vorzusehen. Weiterhin sind andere Steckmechanismen möglich, mit denen die beiden Gehäuseabschnitte miteinander verbunden werden können.

Wie in Fig. 4 dargestellt ist, kann der Gehäuseabschnitt 2 aus einem Material gefertigt sein wie beispielsweise Silikon, das die Geräusche abschwächt, wobei jedes andere Schwammmaterial oder elastisches Material verwendet werden kann, das sich an die Form des Gehörgangs 61 anpasst. Die zwei EEG-Elektroden 3 können beispielsweise als weiche, silberbeschichtete Stoffe ausgebildet sein, die an die Außenfläche des Gehäuseabschnitts 2 angebracht sind. In dieser Konfiguration ist der Gehäuseabschnitt 2 kostengünstig herzustellen und kann nach einmaliger Nutzung mit einer Untersuchungsperson weggeworfen werden.

Wie in Fig. 2 zu erkennen ist, sind die beiden Signalelektroden 3 elektrisch mit den zwei Kontaktringen 4 verbunden, die koaxial in der Buchse 5 ausgebildet sind. Wenn der Gehäuseabschnitt 2 mit dem Gehäuseabschnitt 1 verbunden wird, wird der Kontaktstift 6 in die Buchse 5 eingeführt, wobei die weiteren Kontaktelemente 7 einen elektrischen Kontakt mit den Kontaktringen 4 herstellen. Dadurch sind die EEG-Elektroden 3 mit den Eingängen des Verstärkers 8 verbunden, der vorzugsweise ein vorgespannter Differenzverstärker sein kann. Wie vorher erläutert, wird dann das verstärkte EEG-Signal vom Ausgang des Verstärkers 8 dem Signalprozessor 9 zugeführt, der eine Analog-Digital-Wandlung und eine Signalvorverarbeitung durchführen kann.

Wie in Fig. 2 zu erkennen ist, sind die Längen der Signalleitungen 14, in denen das nicht verstärkte EEG-Signal vorliegt, sehr gering. Dadurch können sich insbesondere bei Anwendung im Zusammenhang mit einer MR-Anlage nicht wie in Fig. 1 gezeigt eine großen Leiterschleifen bilden. In dieser Ausführungsform ist eine mögliche Leiterschleife auf die Eingänge des Verstärkers 8, die Signalleitungen 14 und die Signalelektroden 3 mit den Verbindungselementen 4 und 7 beschränkt. Durch die Verstärkung haben die Signalkomponenten nach dem Verstärker 8 eine niedrige Impedanz und sind somit wenig störempfindlich. Da die verwendeten Wellenlängen bei MR-Anlagen bei ungefähr einem halben Meter liegen, können keine hohen Spannungen induziert werden aufgrund der geringen elektrischen Resonanz.

Somit ist die dargestellte Lösung sehr robust gegenüber externer elektromagnetischer Interferenz, EMI. Weiterhin ist zu erkennen, dass eine dritte Referenzelektrode oder eine vierte Erdungselektrode nicht notwendig ist, da die Vorrichtung elektrisch nicht an eine Erdung gebunden ist, sondern potentialfrei ist. Es sind somit nur zwei Elektroden pro Ohr vorgesehen. In Fig. 2 ist die Vorrichtung nur für ein Ohr dargestellt, selbstverständlich kann die gleiche Vorrichtung für das zweite Ohr verwendet werden. Bei Verwendung von zwei Vorrichtungen können gleichzeitig EEG-Signale vom rechten und vom linken Ohr aufgenommen werden. Hierbei ist eine räumliche und galvanische Trennung zwischen den einzelnen Komponenten vorgesehen, so dass ein elektrischer Stromfluss vom linken Ohr durch das Gehirn zum rechten Ohr vermieden wird. Somit sind die EEG-Signale interferenzfrei und von hoher Qualität und können digital nachverarbeitet werden, um weitere Informationen, aus den Signalen zu erlangen.

Der Gehäuseabschnitt 1 kann auch kompatibel für Verwendung in MR-Anlagen ausgebildet sein, wie es beispielweise in der Patentanmeldung EP 3 315 985 A1 beschrieben ist.

Werden zwei Vorrichtungen an den beiden verschiedenen Ohren verwendet, können gleichzeitig EEG-Signale vom rechten und vom linken Ohr detektiert werden. Für mehrere Nachverarbeitungsverfahren ist es vorteilhaft bzw. notwendig, dass die Signale zeitlich synchronisiert sind. Hierfür kann beispielsweise jede Vorrichtung, insbesondere der Gehäuseabschnitt 1, einen Realzeittaktgeber aufweisen, der beispielsweise in dem Signalprozessor 9 implementiert sein kann. Die Zeitsynchronisation kann hierbei erfolgen, indem die zentrale Verarbeitungseinheit 100 ein Referenzzeitsignal an die beiden Vorrichtungen schickt. Weiterhin kann jedes Datenpaket, das von der Vorrichtung an die zentrale Verarbeitungseinheit geschickt wird, zusätzlich zu den EEG-Signalen einen Zeitstempel der Akquisition beinhalten. Beim Empfang der Daten in der zentralen Verarbeitungseinheit ist somit die genaue Zeit der Akquisition der Signale bekannt mit einer ausreichenden Genauigkeit.

Es wurde gezeigt, dass es möglich ist, die Gehirnaktivität einer Person direkt in Sprache umzusetzen, so dass beispielsweise Computer direkt mit dem Gehirn kommunizieren können. Das Sprechen oder die Vorstellung des Sprechens erzeugt spezifische Aktivitätsmuster im Gehirn. Unterschiedliche Muster ergeben sich ebenfalls, wenn einem Sprechenden zugehört wird. In diesem Zusammenhang sind neuroprothetische Vorrichtungen bekannt, die Sprachdaten rekonstruieren aufgrund der neuronalen Aktivität von Patienten, beispielsweise wenn diese aufgrund einer Lähmung nicht mehr sprechen können. Studien haben gezeigt, dass es möglich ist, aus den Gehirnsignalen Sprache zu rekonstruieren, unter anderem mit sogenannten Deep-Learning-Verfahren und trainierten künstlichen neuronalen Netzen. Die oben beschriebene Vorrichtung kann somit verwendet werden, um EEG-Signale aufzunehmen, wobei dann die zentrale Verarbeitungseinheit 100 ausgebildet ist zu detektieren, ob die Sprachnachrichten der Bedienperson einer MR-Anlage oder voraufgenommene Sprachnachrichten von der Untersuchungsperson korrekt aufgenommen werden und durch das Gehirn der Untersuchungsperson richtig prozessiert werden. Weiterhin ist es möglich, nicht gesprochene, d.h. nur gedachte, Äußerungen in den EEG-Signalen zu detektieren, zu interpretieren und eine Text- oder Sprachnachricht zu erzeugen. Weiterhin kann die Verarbeitungseinheit 100 ausgebildet sein, Sprachnachrichten der Untersuchungsperson oder der Bedienperson zu detektieren und automatisch von einer Sprache in eine andere Sprache zu übersetzen.

Ebenso ist es möglich, dass die Verarbeitungseinheit 100 die EEG-Signale auswertet, um bestimmte Gehirnaktivitäten zu detektieren, die charakteristisch sind für Angst oder Unbehagen der Untersuchungsperson. Das aufgenommene EEG-Signal kann spektral in fünf verschiedene Frequenzbänder unterteilt werden, die Alphawellen von 8 bis 13 Hz, die Betawellen von 13 bis 30 Hz, die Gammawellen von 30 bis 100 Hz, die Thetawellen von 4 bis 7 Hz und die Deltawellen von 0,1 bis 3 Hz. Die Detektion der Betawellen kann als Anzeichen für Unbehagen oder Angst verwendet werden, beispielsweise während einer MR-Untersuchung. Wenn diese Eigenschaften in den EEG-Signalen detektiert werden, kann dies die Verarbeitungseinheit detektieren und die Bedienperson informieren und/oder automatische Aktivitäten starten, die den Patienten beruhigen. Beispielsweise kann eine entspannende Lichttherapie gestartet werden oder entspannende Musik oder Sprachnachrichten von für die Untersuchungsperson bekannten Menschen. Weiterhin kann eine Aromatherapie mit beruhigenden Düften gestartet werden oder es können Videos mit entspannenden Bildern wie Landschaften oder bekannten Gesichtern gestartet werden.

Weiterhin kann die Vorrichtung einen nicht dargestellten Temperatursensor aufweisen, der die Körpertemperatur der Untersuchungsperson im Ohr misst. Dadurch kann die Körpertemperatur überprüft werden, und es kann beispielsweise die Raumbelüftung in Abhängigkeit von der Körpertemperatur gesteuert werden.

Weiterhin ist es möglich, dass die Vorrichtung, beispielsweise der Gehäuseabschnitt 11, einen optischen Sensor aufweist, der die Herzrate misst. Ein derartiger Sensor kann beispielsweise eine Infrarotlichtquelle, beispielsweise eine LED verwenden, und einen Photodetektor, um den pulsierenden Blutfluss unterhalb der Haut zu detektieren. Durch die Bestimmung der Herzrate ist es möglich, die kardiovaskulären Bedingungen der Untersuchungsperson zu überwachen und beispielsweise einen Alarm zu erzeugen, wenn Unregelmäßigkeiten in dem Herzschlag detektiert werden.

Weiterhin ist es möglich, aus den EEG-Signalen zu bestimmen, ob die Untersuchungsperson bei Bewusstsein ist oder nicht. Die oben beschriebenen verschiedenen spektralen Bänder des EEG-Signals haben Einfluss auf die unterschiedlichen Zustände des Bewusstseins. Bei bewusstlosen Personen wird beispielsweise eine Aktivität in den Deltawellen festgestellt. Dadurch kann durch Nachverarbeitung die Verarbeitungseinheit 100 detektieren, ob die Untersuchungsperson bei Bewusstsein ist oder nichts, und gegebenenfalls ein Alarmsignal erzeugen, wenn eine Bewusstlosigkeit detektiert wird.

Ebenso ist es möglich, aufgrund von Stimulationen des Gehirns und der Antworten das Bewusstsein der Untersuchungsperson zu bestimmen. Beispielsweise ist das P300-Signal ein Eventbezogenes Potential, ERP, bekannt wobei der Signalpeak üblicherweise 300 ms nach der Stimulation auftritt. Dies steht im Gegensatz zu den rhythmischen Wellen, die die langfristige Aktivität des Gehirns darstellen. Wenn nun beispielsweise öfters verschiedene Stimulationen dem Patienten zugeführt werden, wie beispielsweise ein akustisches Geräusch oder ein visueller Stimulus wie das Licht einer Lampe, so kann daraus aus den EEG-Signalen abgeleitet werden, ob die Person bei Bewusstsein ist oder nicht. Wenn nur eine geringe ERP-Antwort detektiert wird oder überhaupt keine, so ist dies ein sicheres Anzeichen, dass die Untersuchungsperson nicht bei Bewusstsein ist.

Ebenso ist es möglich, durch die Nachverarbeitung der EEG-Daten zu detektieren, ob die Untersuchungsperson schläft oder ob die Untersuchungsperson einen anderen bestimmten Mechanismus der Gehirnfunktion durchführt. Durch die Analyse der Frequenzkomponenten des EEG-Signals ist es möglich, zu überprüfen, ob die Untersuchungsperson schläft. Dies ist insbesondere vorteilhaft bei der Anwendung im Zusammenhang mit der funktionellen MR-Bildgebung, da dort verschiedene Stimuli bei schlafenden oder wachen Untersuchungspersonen appliziert werden und die Gehirnaktivität mithilfe der MR-Bilder detektiert wird.

Weiterhin ist es möglich, mithilfe der EEG-Signale oder Gehirnaktivität eine Untersuchungsperson zu identifizieren. Somit können die EEG-Biometriedaten für die Patientenauthentifizierung verwendet werden. Hierfür muss die Untersuchungsperson beispielsweise eine vordefinierte Tätigkeit ausführen während der Aufnahme der EEG-Signale. Dies kann beispielsweise das innere Summen eines Lieds oder eine simulierte Bewegung einer bestimmten Extremität sein. Die aufgenommenen Daten werden in verschiedene Segmente unterteilt, von denen jeweils Merkmale extrahiert werden, wie beispielsweise autoregressive Koeffizienten, die spektrale Energiedichte und die Gesamtenergie in den verschiedenen EEG-Frequenzbändern. Diese Merkmale können zu einem Merkmalsvektor kombiniert werden, der dann verwendet wird durch eine trainierte Support-Vektor-Maschine, SVM, für die automatische Authentifizierung der Untersuchungsperson.

Zusammenfassend ist festzustellen, dass die erfindungsgemäße Vorrichtung die Möglichkeit der induktiven oder kapazitiven Kopplung von Störsignalen minimiert durch die Verwendung von sehr kurzen Signalleitungen und der drahtlosen Übertragung der EEG-Signale.

## Patentansprüche

1. Vorrichtung zum Messen von EEG-Signalen in einem Ohr einer Untersuchungsperson, aufweisend:
- ein Gehäuse,
- zwei auf einer Außenseite des Gehäuses angeordneten Signalelektroden (3) zur Aufnahme von EEG-Signalen von einer Innenfläche des Ohres,
wobei in dem Gehäuse vorgesehen sind:
- ein Verstärker mit zumindest zwei Eingängen zur Verstärkung der mit den Signalelektroden aufgenommenen EEG-Signalen,
- eine Sende-/Empfangseinheit, die ausgebildet ist, EEG-Daten nach der Verstärkung durch den Verstärker drahtlos an eine zentrale Verarbeitungseinheit zu übertragen,
- zwei Signalleitungen, über die die EEG Signale der Signalelektroden an den Verstärker übertragen werden, wobei jede der zwei Signalleitungen nicht länger als 2 cm ist, vorzugsweise nicht länger 1 cm.

2. Vorrichtung nach Anspruch 1, **dadurch gekennzeichnet, dass** das Gehäuse einen ersten Gehäuseabschnitt (2) und einen zweiten Gehäuseabschnitt (1) aufweist, die lösbar miteinander verbindbar sind, wobei der erste Gehäuseabschnitt die beiden Signalelektroden aufweist, und der zweite Gehäuseabschnitt den Verstärker und die Sende-/Empfangseinheit aufweist.

3. Vorrichtung nach Anspruch 2, **dadurch gekennzeichnet, dass** der erste Gehäuseabschnitt (2) ein erstes Verbindungselement (5) und der zweite Gehäuseabschnitt (1) ein zweites komplementäres Verbindungselement (6) aufweist, wobei das erste und zweite komplementäre Verbindungselement ausgebildet sind, den ersten Gehäuseabschnitt (2) und den zweiten Gehäuseabschnitt (1) lösbar miteinander zu verbinden, wobei das erste Verbindungselement (5) zwei Kontaktelemente (4) aufweist, die in Verbindung mit den zwei Signalelektroden stehen, wobei das zweite Verbindungselement zwei weitere Kontaktelemente (7) aufweist, die jeweils in elektrischer Verbindung mit dem Verstärker stehen, wobei jede der zwei weiteren Kontaktelemente (7) im verbundenen Zustand der beiden Gehäuseabschnitte jeweils mit einem der zwei Kontaktelemente (4) verbunden ist.

4. Vorrichtung nach Anspruch 3, **dadurch gekennzeichnet, dass** das erste Verbindungselement (5) als Kontaktbuchse und das zweite komplementäre Verbindungselement (6) als Kontaktstift ausgebildet sind, wobei die beiden weiteren Kontaktelemente auf dem Kontaktstift ausgebildet sind, und die Kontaktelemente des ersten Verbindungselements als Kontaktringe ausgebildet sind, in die der Kontaktstift im verbundenen Zustand eingeführt ist, und welche die auf dem Kontaktstift ausgebildeten weiteren Kontaktelemente kontaktieren.

5. Vorrichtung nach einem der vorstehenden Ansprüche, **dadurch gekennzeichnet, dass** in dem Gehäuse weiterhin ein Signalprozessor vorgesehen ist, der ausgebildet ist, ein verstärktes EEG-Signal nach Verstärkung im Verstärker zu empfangen, das verstärkte EEG-Signal zu filtern und einer Analog-Digitalwandlung durchzuführen.

6. Vorrichtung nach einem der vorstehenden Ansprüche, weiterhin **gekennzeichnet durch** einen optischen Sensor, der ausgebildet ist, die Herztätigkeit der Untersuchungsperson zu detektieren.

7. Vorrichtung nach einem der vorstehenden Ansprüche, **dadurch gekennzeichnet, dass** das Gehäuse Außenmaße aufweist, die 5 cm, bevorzugt 3 cm nicht überschreiten.

8. Vorrichtung nach einem der vorstehenden Ansprüche, **dadurch gekennzeichnet, dass** sie nur zwei Signalelektroden aufweist.

9. System zur Verarbeitung von EEG-Signalen, das eine Vorrichtung nach einem der Ansprüche 1 bis 8 aufweist und eine zentrale Verarbeitungseinheit aufweist, die ausgebildet ist, die von der Sende-/Empfangseinheit drahtlos übertragenen EEG-Daten zu empfangen und weiterzuverarbeiten.

10. System nach Anspruch 9, **dadurch gekennzeichnet, dass** das System jeweils zwei Vorrichtungen nach einem der Ansprüche 1 bis 8 aufweist, eine für jedes Ohr der Untersuchungsperson, wobei die zentrale Verarbeitungseinheit ausgebildet ist, ein Referenzzeitsignal für die beiden zwei Vorrichtungen zu erzeugen, mit dem die EEG-Daten der beiden Vorrichtungen zeitlich synchronisiert werden.

11. System nach Anspruch 9 oder 10, **dadurch gekennzeichnet, dass** die zentrale Verarbeitungseinheit ausgebildet ist, zumindest einen der folgenden Schritte durchzuführen:
- anhand der EEG-Daten zu detektieren, ob eine Sprachnachricht, die an die Untersuchungsperson gerichtet wurde, von der Untersuchungsperson richtig verarbeitet wurde,
- aus den EEG-Daten unausgesprochene Gedanken zu extrahieren und in eine Sprachnachricht umzuwandeln,
- anhand der EEG-Daten zu detektieren, ob die Untersuchungsperson Angst hat oder Unbehagen verspürt,
- anhand der EEG-Daten zu detektieren, ob die Untersuchungsperson das Bewusstsein verliert,
- anhand der EEG-Daten zu detektieren, ob die Untersuchungsperson schläft,
- anhand der EEG-Daten eine Identität der Untersuchungsperson zu detektieren.

12. System nach einem der Ansprüche 9 bis 11, wobei die zentrale Verarbeitungseinheit eine zentrale Steuereinheit einer MR-Anlage ist.
